# EUROPEAN PATENT SPECIFICATION

(11) **EP 2 983 651 B1**
(45) Date of publication and mention of the grant of the patent: **26.12.2018**
(21) Application number: 13718529.4
(22) Date of filing: 12.04.2013
(51) Int. Cl.: A61K 9/68, A61K 9/20, A61K 31/722, A61P 3/12, A61P 13/12

(54) **COMPOSITIONS COMPRISING A MIXTURE OF ELASTOMERS AND CHITOSAN AND USE THEREOF FOR TREATING DISORDERS OF PHOSPHOROUS MINERAL METABOLISM**
ZUSAMMENSETZUNGEN MIT EINER MISCHUNG AUS ELASTOMEREN UND CHITOSAN SOWIE VERWENDUNG DAVON ZUR BEHANDLUNG VON LEIDEN DES PHOSPHORMINERALMETABOLISMUS
COMPOSITIONS COMPRENANT UN MÉLANGE D'ÉLASTOMÈRES ET DE CHITOSAN ET SON UTILISATION POUR TRAITER DES TROUBLES DU MÉTABOLISME DES MINÉRAUX PHOSPHORÉS

(43) Date of publication of application: 17.02.2016
(73) Proprietor: ExPharma Limited, Fulham Broadway London SW6 1ENUK (GB)
(72) Inventor: COMELLI, Maria Cristina, 35127 Padova (IT); RAMPAZZO, Paolo, I-35128 Padova (IT)
(74) Representative: Gervasi, Gemma
(86) International application number: PCT/EP2013/057680
(87) International publication number: WO 2014/166545

(56) References cited:
- WO-A1-2012/143011
- SAVICA V ET AL.: "Salivary phosphate-binding chewing gum reduces hyperphosphatemia in dialysis patients", JASN, vol. 20, 2009, pages 639-644, XP002717466, cited in the application
- MAGGI L ET AL: "Preparation and evaluation of release characteristics of 3TabGum, a novel chewing device", EUROPEAN JOURNAL OF PHARMACEUTICAL SCIENCES, ELSEVIER, AMSTERDAM, NL, vol. 24, no. 5, 1 April 2005 (2005-04-01), pages 487-493, XP027803771, ISSN: 0928-0987 [retrieved on 2005-04-01]

## Description

### FIELD OF INVENTION

The present invention relates to compositions comprising a mixture of elastomers and chitosan suitable to reduce the serum levels of phosphorous compounds, in particular phosphates, by *in situ* up-taking the salivary phosphorous compounds in the pH range of 6.5-7.4, where the chitosan is the phosphorous binding agent and the same is not released by the compositions themselves. The present invention further relates to the use of said compositions for preventing or treating disorders of phosphorous mineral metabolism associated to an increased salivary phosphorous levels.

### BACKGROUND OF THE INVENTION

Phosphorous (P) metabolism disorders are regarded as non-traditional risk factors for increased cardiovascular events and mortality in the general population, in pre-dialysis Chronic Kidney Disease (CKD) subjects, and even more significantly in End Stage Renal Disease (ESRD) patients undergoing dialysis. It has been recently suggested that an increased phosphate content into the human saliva reflects, and could even anticipate, an overt phosphorous metabolism disorder. Early stage CKD subjects present with detectable increased salivary phosphate content when their serum phosphorous level falls into the range of 3.5 to 4.5 mg/dL, so that they are still regarded as normophosphatemic, despite the serum levels of other markers of phosphorous metabolism can be altered, as it could be the case of FGF-23, PTH and cholecalciferol levels [Komaba H et al. Treatment of chronic kidney disease-mineral and bone disorder (CKD-MBD). Internal Medicine 2008;47:989-994*].* These evidences not only suggest that the increased salivary phosphate content could be a possible early marker of altered P metabolism, but also supports the concept that salivary phosphate contribute to P mass balance by its ingestion [Tonelli M et al. Oral phosphate binders in patients with kidney failure. New Engl J Med 2010; 362:1312-24]. Actually, food intake is the only source of phosphorous to the body, being absorbed at the intestinal level in the order of 60% to 80-100% of its content into the enteric fluids, in normal and diseased conditions respectively. In biological fluids, phosphorous is found as HPO₄²⁻, H₂PO₄⁻ and PO₄³⁻, commonly named as "phosphates". Phosphorous, phosphorous compounds and phosphates (P) are considered as interchangeable into the medical literature, and, therefore, these terms are herein used as synonymous. Phosphates are promptly bioavailable for intestinal absorption [Uribarri J. Phosphorus homeostasis in normal health and in chronic kidney disease patients with special emphasis on dietary phosphorus intake. Seminars in Dialysis 2007;20:295-301*].*

Dietary P enters the enteric fluids in form of organic phosphoproteins and phospholipids, and as phosphates mainly via food additives. The formers are amenable to dietary phosphate-restriction via a reduced protein intake, medically advised in CKD subjects at 1.0-1.2 g/day/Kg body weight (b.w.) and resulting in 700-1000 mg/die phosphate, liberated into the gut from digestive processes *[*Takeda E et al., Phosphate restriction in diet therapy. Nutrition and Kidney Disease: a new era 2007;155:113-124*].* The latters frequently escape any efficient dietary restriction because of no regulatory obligations currently in place to have their content fully declared in food labeling. Therefore, it is the excess of bioavailable phosphates to be currently envisaged as responsible for the maintenance of a positive P mass balance in body fluids [Uribarri J., Phosphorus additives in food and their effect in dialysis patients. Clin J Am Soc Nephrol 2009;4:1290-1292*; Uribarri J., 2007, ref*. *cit*.*]*.

As a proof of concept, a dietary intervention aimed to restrict the intake of phosphate-enriched food additives by avoidance of processed foods was demonstrated to be as effective as small doses of intestinal phosphate binders, i.e. drugs usually prescribed to treat hyperphosphatemia by reduction of intestinal phosphorous absorption. Interestingly, the food additive-restricted diet brought a 0.6 mg/dL decrease in serum P levels over three months, being such decrease clinically relevant in reducing the risk of cardiovascular mortality [Sullivan C et al., Effect of food additives on hyperphosphatemia among patients with End-Stage Renal Disease. A randomized controlled trial. JAMA 2009, 301: 629-635]. WO 2006/061336 [Savica V, Oral composition for absorption of phosphorus compounds, 15 June 2006] provides pharmaceutical compositions to be administered by oral route in fasting conditions to absorb phosphorous from fluids of the enteric tract, especially from saliva. Such absorption results from the continuous slow release of the phosphate binders, including chitosan, into the gastrointestinal tract.

Accordingly, the compositions disclosed by WO 2006/0613336 are claimed for use in the treatment of hyperphosphatemia in fasting conditions. In clinically exploiting these compositions, Savica V et al. [Savica V et al., Salivary phosphate-binding chewing gum reduces hyperphosphatemia in dialysis patients. JASN, 2009; 20: 639-644] reported the beneficial effect of a chewing-gum, acting as a slow release delivery system for the chitosan as a phosphorous binding agent, in the reduction of hyperphosphatemia. A reduction in the amount of salivary phosphates was also observed. Serum and salivary P reduction was discussed by the Authors as related to the chitosan being released into the enteric fluids, where the chitosan dissolves and is retained up to 40 hours from administration. Savica V et al. reported that the released chitosan prevents the binding capability of the phosphate binders (medically prescribed to be administered at meals for the reduction of intestinal P absorption) from being saturated by the salivary phosphates collected into the enteric fluids.

The same approach of Savica V. et al. drawn to treat hyperphosphatemia is disclosed in WO 2012/143008 *[Steenberg LCK Reduction of the level of free phosphorus compounds in the digestive juice, 26 October 2012]* and WO 2012/1430011 [*Steenberg LCK et al. Chewing gum comprising chitosan for use in reduction of the level of free phosphorus compounds in the digestive juice, 26 October 2012].* In fact, WO 2012/143008 claims slow release oral compositions, preferably chewing gum, comprising the phosphorous binding agent chitosan and an organic acid, in particular ascorbic acid, for reducing free phosphorous compounds in digestive juices, where the organic acid is combined to chitosan for improving the release thereof from the compositions. WO 2012/143011 discloses the same slow release oral compositions of chitosan, again preferably form of chewing gum, where is shown the capability of the organic acid ascorbic acid to improve the release of chitosan from the compositions.

However, Savica V et al. have demonstrated in the clinical investigation previously mentioned that, in dialyzed ESRD patients, an averaged 70 mg/dL of salivary phosphates are estimated to contribute for not less than 250-350 mg/day of promptly bioavailable phosphates [*Savica V et al*., *2009*, *ref*. *cit*.]. Upon swallowing, the salivary phosphates are collected into the enteric fluids and undergo intestinal absorption, being looped again into the saliva because of re-excretion at the salivary glands level. Therefore, the excess of salivary phosphate content in ESRD patients is continuously supplying a secondary phosphate intake, spoiling the body's attempt to rid the toxic phosphorous compounds.

Therefore, the need for compositions comprising the chitosan as the phosphorus binder not to be released into the gastrointestinal tract, and thus to act exclusively as a salivary phosphate binder, irreversibly reducing the intake of salivary phosphates before they are ingested by swallowing, is still felt in the field, since this approach can be the only effective solution for treating disorders of phosphorous mineral metabolism, which are associated with serum P levels approaching the upper limits of normophosphatemia (serum P≥3.5 mg/dL), and particularly in hyperphosphatemia (serum P>4.5 mg/dL), as it is the case of early and advanced Chronic Kidney Disease, hyperparathyroidism, hypertension, diabetes, cardiovascular disease associated with calcification of the media and intima wall of arteries, renal osteodistrophy [recently renamed Mineral Bone Disorder (MBD)].

The restriction of salivary phosphate intake could be supportive to the primary dietary management of phosphates via protein-restricted diet in P mineral disturbances/disorders. The prior art in the field does not technically solve the issue in full, being the oral compositions described in the prior art only partially binding the salivary phosphates into the oral cavity.

### SUMMARY OF THE INVENTION

The primary purpose of the present invention is, therefore, to solve the aforesaid problem and to provide compositions aimed to: a) restrict the salivary phosphates intake, otherwise ingested when the saliva is swallowed, by binding said salivary phosphates *in situ* in the oral cavity by means of an effective phosphorous binding agent incorporated into a suitable composition; and b) do not allow said phosphorous binding agent to be dissolved and released from the composition itself, either before and after having formed a stable complex with the salivary phosphates.

In order to fulfill the purposes pursued with the invention, the compositions to be preferred are chewable compositions, and preferably chewing gums, where the phosphorous binding agent is incorporated and entrapped in a chewable gum base, and by itself is insoluble at the oral pH and with a hindrance and a structural conformation suitable to render the same un-releasable from the chewable gum base.

Surprisingly, as detailed in the following description, compositions comprising a gum base consisting of synthetic elastomers and mixtures thereof and a chitosan have been proved to fulfill the dual requirements of no chitosan release and phosphorous binding.

Therefore, it is an object of the present invention to provide a chewable composition comprising a gum base consisting of synthetic elastomers and mixtures thereof and a chitosan, wherein the chitosan has a viscosity (in 1% acetic acid) in the range from at least 10 to 100 mPa.s and a deacetylation degree (DAC) of not less than 90% and is retained and/or not released from the composition and is capable to bind salivary phosphorous compounds by up-taking the same *in situ* in the mouth cavity, for use to reduce serum levels of phosphorous compounds in disorders of phosphorous mineral metabolism. The disorders of phosphorus mineral metabolism are preferably phosphorous mineral metabolism disorders associated with a detectable increased salivary phosphate content.

These and other objects as well as features and advantages of the present invention will be better understood from the detailed description set forth below and from the preferred embodiments which are given for illustration and not limiting purposes.

### DETAILED DESCRIPTION

### Definitions and abbreviations

The wordings "phosphorous binding agent" or "phosphate binding agent" or "phosphorous binder" or "phosphate binder" will be used in the whole description to indicate compounds capable to bind phosphorous compounds present in the saliva at pH values of at least or higher than 6.5 and regarded as promptly available for intestinal absorption upon ingestion, such as HPO₄²⁻, PO₄³⁻ and H₂PO₄⁻.

The phosphorous binding agent is the polycationic polysaccharide chitosan, derived from the deacetylation of either animal or vegetal chitin. The chitosans of animal or vegetal origin are linear co-polymers composed of randomly distributed [beta]-(1-4)-linked D-Glucosamine and N-acetyl-D-Glucosamine, but the chitosan from vegetal sources can additionally contains β-glucan (a backbone of glucose residues linked together by 1-3 linear β-glycosidic bonds with β1,4 or β1,6 branches). The chitosans suitable for the chewable compositions according to the present invention are at least of food-acceptable grade.

With the word "elastomer(s)" are meant at least food-acceptable grade synthetic rubbers, and mixture thereof, selected among those characterised by not requiring melting for the addition of the other components of the composition, and by suitability for cold compression.

The synthetic elastomers are without limitation: isobutylene-isoprene copolymers, polyisobutylene, polyisoprene, styrene-butadiene copolymers, polyethylene, polyvinyl acetate, polyvinyl acetate/laurate copolymers and the like and mixtures thereof.

### Description

The present invention concerns oral chewable compositions aimed to the restriction of salivary phosphate intake, before the phosphates are ingested with the swallowing of the saliva. The purpose of the present invention requires that such compositions simultaneously: a) do not allow the chitosan to be released from the proper composition by dissolution and/or suspension when exposed to aqueous solutions in the range of pH 6.0-7.4 even for a prolonged chewing time, and b) do allow the composition to be permeated by a phosphoric aqueous solution in order to permit the occurrence of the phosphate binding by P-binding agent chitosan.

As detailed hereafter, during the assessment of oral compositions aimed to pursue the dual performance of no chitosan release and P binding for the restriction of salivary phosphate intake before ingestion, different gum bases were used. Surprisingly, no composition other than the one object of the present invention was able to simultaneously avoid the release of chitosan and allow significant binding of the phosphates, further retaining the P-chitosan complex into the chewing-gum. Therefore, the present invention provides a chewable composition, preferably a chewing-gum, comprising a gum base consisting of synthetic elastomers and/or mixtures thereof and a chitosan, wherein the preferred gum base is a mixture of synthetic elastomers not requiring melting for the addition of the other components for the preparation of the compositions and suitable for cold compression. The most preferred gum base is, therefore, a mixture of synthetic elastomers comprising at least butyl rubber (isobutylene-isoprene copolymers) and polyisobutylene polymers.

The phosphate binder chitosan is either of animal or vegetal origin and irrespective of its origin, the chitosan has a viscosity (in 1% acetic acid) in the range from at least 10 to 100 mPa.s, preferably from 70 to 90 mPa.s, and a deacetylation degree (DAC) of not less than 90%, and preferably equal or higher than 95%.

For the purposes of the present invention these features of chitosan are particularly favourable, being the chitosans having these characteristics insoluble at the salivary pHs from 6.0 to 7.4 and capable to bind phosphates as shown hereinafter.

Another feature of the chewable compositions object of the invention which has proved to be substantial is the ratios by weight between the two components gum base and chitosan. The ratios effective in retaining the chitosan in the composition and avoiding its release are in the range from 1:4 (gum base : chitosan) and 4:1 (gum base: chitosan) (0.25-1.0) and preferably the ratio is 1 to 1 (gum base : chitosan).

Furthermore the amounts of the gum base and chitosan respect the total weight of the chewable composition are respectively from 5 to 6 % (gum base) and from 1 to 20% (chitosan).

In fact, at pH higher than 6.5, corresponding to the human salivary pH upon stimulation by chewing, such compositions were found not to allow chitosan dissolution and release from the chewing gum over a 60 minutes chewing time. The dissolution test in chewing machine using artificial saliva containing amounts of phosphate similar to either human normophosphoric or hyperphosphoric saliva, demonstrated that such compositions do not release the chitosan, while allowing the chitosan to act as a phosphate binder. Therefore, such compositions could be profitably used to restrict the amount of phosphates in the saliva, thus reducing the intake of salivary phosphates. Further, in the specific conditions of use, such compositions were demonstrated not to allow the formed phosphate-chitosan complex to be released from the gum. Therefore, such compositions can be profitably used to restrict phosphate intake from saliva, before saliva is ingested and collected into the enteric fluids.

Therefore, in an embodiment of the present invention the chewable compositions comprise a gum base (a) and chitosan (b) in a ratio in the range from 1(a):4(b) to 4(a):1(b), where (a) is the butyl rubber/polyisobutylene; and (b) is chitosan of animal and/or vegetal origin, selected from chitosans having a viscosity (in 1% acetic acid) of from 10 to 100 mPa.s and a DAC of at least 90%.

In another embodiment of the present invention the chewable compositions comprise a mixture of gum base (a) and chitosan (b) in a ratio in the range from 1(a):4(b) to 4(a):1(b), where (a) is the butyl rubber/polyisobutylene; and (b) is chitosan of animal and/or vegetal origin, selected from chitosans having a a viscosity (in 1% acetic acid) from 70 to 90 mPa.s and a DAC not less than 90% and preferably equal or higher than 95%.

In a further embodiment of the present invention the chewable compositions comprise a mixture of gum base (a) and chitosan (b) in a ratio in the range from 1(a):4(b) to 4(a):1(b), where (a) is the butyl rubber/polyisobutylene; and (b) is chitosan of animal and/or vegetal origin, selected from chitosans having a a viscosity (in 1% acetic acid) from 70 to 90 mPa.s and a DAC equal or higher than 95%.

It is yet an embodiment of the present invention chewing gum compositions comprising a gum base (a) and chitosan (b) in a ratio from 1(a):1(b) where (a) is the butyl rubber/polyisobutylene; and (b) is chitosan of animal and/or vegetal origin, selected from chitosans having a viscosity (in 1% acetic acid) of at least from 10 to 100 mPa.s, and a DAC of at least 90%.

It is another embodiment of the present invention chewing gum compositions comprise a gum base (a) and chitosan (b) in a ratio 1(a):1(b), where (a) is the butyl rubber/polyisobutylene; and (b) is chitosan of animal and/or vegetal origin, selected selected from chitosans having a viscosity (in 1% acetic acid) from 70 to 90 mPa.s, and a DAC of at least 90%.

It is a further embodiment of the present invention chewing gum compositions comprise a gum base (a) and chitosan (b) in a ratio 1(a):1(b), where (a) is the butyl rubber/polyisobutylene; and (b) is chitosan of animal and/or vegetal origin, selected selected from chitosans having a viscosity (in 1% acetic acid) from 70 to 90 mPa.s, and a DAC equal or higher than 95%.

These compositions can be profitably used to reduce salivary phosphate intake before its ingestion, as a complement to a medically advised dietary requirement for the reduction of phosphate intake from any source.

The compositions of the present invention can be presented in the form of uncoated chewing gums, center-fill chewing gum tablets, or as the inner layer (gum core) of a multilayered cold compressed chewing gum.

The chewable compositions of the present invention can further comprise plasticizers, resins, anti-oxidants, sweeteners, excipients and flavours, without impairment of the expected performances. These components can be selected from those known to one with an ordinary skill in the art with the provision that said components fulfill a necessary requirement of not interfering on oral pH by acidification.

As a title of example the plasticizers can be selected from the group consisting of vegetal natural rubbers, glycerol ester of partially dimerized rosin, glycerol ester of partially hydrogenated gum of wood rosin, glycerol ester of polymerized rosin, glycerol ester of gum rosin, glycerol ester of tall oil rosin, glycerol ester of wood rosin, lanolin, methyl ester of rosin (partially hydrogenated), pentaerythritol ester of partially hydrogenated gum or wood rosin, pentaerythritol ester of gum or wood rosin, rice bran wax, stearic acid, sodium, potassium and magnesium stearates, glycerol monostearates, hydrogenated oils, acetylated monoglycerides, soy lecithin, and mixtures thereof. The vegetal natural rubber are the preferred plasticizers and are, for example without limitation, selected from chicle gum, natural rubber, gutta-percha, and the like and mixtures thereof.

In the chewable compositions of the invention, examples of resins can be selected from the group consisting of polyvinylacetate, terpene resins synthetic resin consisting of polymers of α-pinene, β-pinene, and/or dipentene; and natural resin consisting of polymers of α-pinene and mixtures thereof.

In such compositions, antioxidants can be selected for instance from the group consisting of butylated hydroxyanisole, butylated hydroxytoluene, propyl gallate and mixtures thereof.

As for the sweeteners, the preferred ones for the chewable compositions of the invention are those with low glycemic index and can be selected from the group consisting of isomalt, sorbitol, sodium saccharin, acesulfame K, sugar esters and mixtures thereof.

Other components preferred are food grade excipients selected from the group consisting of silicon dioxide, talc and mixtures thereof.

As examples, but not limited to, the preferred flavours can be menthol, mint/eucalyptol, licorice, and mixtures thereof.

The chewable compositions of the invention have proved to be suitable for treating P metabolism disturbances/disorders reflected, or even anticipated, by an increase in salivary phosphate content characterized by a serum phosphorous level equal or higher than 3.5 mg/dL and a salivary phosphorous content of at least and not less than 17 mg/dL.

These phosphorous metabolism disorders can occur in early and advanced stages of chronic kidney disease, diabetes, hypertension, renal osteodistrophy or mineral bone disorders, hyperparathyroidism and cardio-vascular diseases characterized by an extra-intimal calcification, particularly of the medial wall of the arteries.

The results herein described demonstrate that the compositions according to the present invention fulfil the purposes of the present invention. Further implementation or adaptations as well as embodiments readily apparent to those skilled in the art are to be considered within the scope of the present invention.

The description of chewable compositions made of elastomers other than the mixture selected in the present invention are also reported; they have to be considered as comparative examples so as the purpose of the present invention is concerned. The preferred embodiments are given for illustration and not limiting.

### EXAMPLES

### Example 1: characterization of chitosan and preparation of chewable compositions comprising butyl rubber/polyisobutylene elastomers and chitosan

Hereinafter the studies of characterization of chitosan usable for the chewable compositions object of the invention are reported.

### Solubility of animal and vegetal chitosans at salivary pHs

In order to select animal or vegetal chitosans to remain insoluble into the gum matrix still retaining capability for P binding when exposed to a phosphoric aqueous solution at pH close to or above 6.5, as per the human saliva under stimulated conditions by chewing, different samples of commercially available chitosans (Table 1, identified by acronyms) were tested.

**Table 1 - characteristics of chitosan**

| **ID** | **ORIGIN/GRADE** | **VISCOSITY* (mPa.s)** | **MW (KDa)** | **DAC %** |
|---|---|---|---|---|
| LK | Animal/lab reference | < 200 | ≅400 | 75-80 |
| MK | Animal/lab reference | 200-400 | 400-500 | 75-80 |
| HK | Animal/lab reference | >400 | > 500 | 75-80 |
| K1 | Animal/food | 50-200 | 150-400 | >95 |
| K2 | Animal/food | ≤ 100 | < 200 | 90 |
| A | Vegetal/food | 14 | 75 | 76 |
| B | Vegetal/food | 38 | 108 | 78 |
| C | Vegetal/food | 22 | 78 | 80 |
| D | Vegetal/food | 10 | 42 | 90 |
| E | Vegetal/food | 4 | 17.6 | 84 |

| | | | | |
|---|---|---|---|---|
| **1% in acetic acid* | | | | |

Depending on the physico-chemical characteristics of the chitosan used, significant differences in chitosan solubility can occur in different salivary conditions (unstimulated, stimulated, after stimulation). Therefore chitosans in Table 1 were tested to highlight the effects on water solubility of viscosity and DAC. Different sample amounts (1, 5, 10 mg) were suspended in 1 ml of PBS at pH 6.0, 6.3, 6.5, 7.4. In none of these conditions, the samples were fully solubilised. The supernatants were collected and analyzed for their chitosan content. Solubilized animal chitosan concentrations (in µg/ml) in the various conditions are summarised in Table 2. In the range of pH considered, only LK sample (viscosity ≤ 200 mPa.s and DAC 75-80%) showed a change in solubility, whereas the others showed solubility substantially constant and independent from the amount of chitosan in contact with the liquid. A dissolution experiment on K2 (≤ 100 mPa.s, DAC 90%) at 1 mg/ml in 1% acetic acid and pH adjustment with 5% NaOH, demonstrated its precipitation to occur in a narrow range of pH, between 5.35 and 5.74.

**Table 2- Solubility (in µg/ml) of chitosan samples in the pH range 6.0- 7.4**

| **Sample** | **PBS pH 6.0** | **PBS pH 6.3** | **PBS pH 6.5** | **PBS pH 7.4** |
|---|---|---|---|---|
| LK 1 mg/ml | 111 | 91 | 86 | 42 |
| LK 5 mg/ml | 146 | 97 | 107 | 46 |
| LK 10 mg/ml | 172 | 103 | 107 | 45 |
| HK 1 mg/ml | 58 | 66 | 71 | 61 |
| HK 5 mg/ml | 67 | 68 | 59 | 67 |
| HK 10 mg/ml | 58 | 39 | 55 | 68 |
| K1 1 mg/ml | 61 | 66 | 63 | 57 |
| K1 5 mg/ml | 66 | 65 | 68 | 75 |
| K1 10 mg/ml | 64 | 46 | 75 | 88 |
| MK 1 mg/ml | 38 | 41 | 54 | 46 |
| MK 5 mg/ml | 42 | 40 | 50 | 48 |
| MK 10 mg/ml | 39 | 37 | 53 | 45 |
| K2 1 mg/ml | 40 | 44 | 43 | 61 |
| K2 5mg/ml | 54 | 37 | 50 | 61 |
| K2 10 mg/ml | 58 | 41 | 49 | 67 |

A comparison of animal and vegetal chitosan water solubility in PBS (phosphate concentration 0.55 g/L) at pH 6.5 and 7.4, and in hyperphoshoric artificial saliva (H, Phosphate concentration: 2.24 g/L) at pH 7.4 is reported in Table 3. Clearly, all tested chitosans are poorly soluble in a non-acidic aqueous solution, particularly at pH≥6.5 as per human saliva upon stimulation. However, the vegetal chitosan D (viscosity approx 10 mPa.s and DAC 90%) presented with a behavior similar to K2. Particularly at pH 6.5, simulating salivary conditions when chewing begins, chitosans with a viscosity not more than 100 mPa.s and DAC not less than 90% remain substantially insoluble and even more do so at increasing pH. This evidence is the ground for selecting animal or vegetal chitosan with viscosity of 10 to 100 mPa.s, preferably 70-90 mPa.s, and of a DAC not less than 90%, and preferably equal or higher than 95%, to be used in the compositions of the present invention in order to secure no dissolution and release.

**Table 3 - solubility of chitosan samples in PBS**

| **ID** | **PBS pH 6.5 µg /ml** | **PBS pH 7,4 µg /ml** | **H pH 7.4 µg /ml** |
|---|---|---|---|
| **K2** | **26.1** | 22.0 | 34.4 |
| A | 49.8 | 33.7 | 26.4 |
| B | 61.4 | 21.3 | 8.2 |
| C | 51.1 | 18.7 | 29.5 |
| **D** | **22.0** | 14.9 | 8.9 |
| E | 50.1 | 60.2 | 9.2 |

### Phosphate complexation at salivary pHs

The phosphate binding capacity of animal and vegetal chitosans was assessed in PBS (phosphate concentration 0.55 g/L), at pH 7.4. To this end, nominal chitosan concentrations of 5-100 mg/ml were used. The suspensions so obtained were incubated overnight at 37 °C under stirring (200 rpm). Liquid and solid phases were separated by centrifugation (5 minutes at 11000 rpm) in order to collect the chitosan into the precipitate, and analysed separately. Solid phase (the precipitate) was dissolved in the minimum volume of 1% acetic acid (20 µl-1 ml) and phosphate bound to chitosan measured. The phosphate bound to chitosan is measured after washing with water of the precipitate, which includes the P bound to chitosan and the phosphate physically entrapped into the solid phase. The amount of phosphate in different chitosan samples (100 mg/ml) is presented in Table 4 where the determination of residual phosphate in solution (supernatant), phosphate entrapped in the solid phase (precipitate+washing) and phosphate bound to chitosan after washing (phosphate content of PBS prior to exposure to chitosan: 0.55 mg/ml) (Mean±SD, n=3 repetitions) are summarized. If not differently specified, percentage refers to the amount of phosphate removed from the phosphoric solution.

**Table 4 - phosphate residual content determination**

| **Sample (mg/ml)** | **Supernatant** (PBS) | **Precipitate+Washing** | **Washing** (H₂O milliQ) | **Chitosan** (in the precipitate) |
|---|---|---|---|---|
| | *Residual phosphate* mg | *Total Phosphate removed from PBS* mg (%) | *Phosphate entrapped* mg (%)* | *Phosphate bound* mg (%) |
| K1 100 mg/ml *50-200 mPa.s, DAC >95%* | 0.344±0.001 | 0.31±0.046 (56.4) | 0.059±0.002 (19) | 0.251±0.044 (46) |
| K2 100 mg/ml *≤100 mPa.s, DAC 90%* | 0.332±0.002 | 0.289±0.014 (52.5) | 0.085±0.001 (29) | 0.204±0.013 (37) |
| MK 100 mg/ml *200-400 mPa.s, DAC 75-80%* | 0.256±0.008 | 0.292±0.023 (53) | 0.096±0.001 (33) | 0.196±0.022 (36) |
| D 100 mg/ml *10 mPa.s, DAC 90%* | 0.415 ± 0.09 | 0.237 ± 0.02 (43) | 0.040±0.02 (17) | 0.197±0.02 (36) |

| | | | | |
|---|---|---|---|---|
| *percentage calculated on the precipitate*+*washing quantity* | | | | |

Since washing with water extracts the phosphate not bound but physically entrapped, the results indicate: i) the formation of a complex at the liquid-solid interface during contact of a phosphate-containing solution with chitosan powder, ii) once formed, the phosphate-chitosan complex is stable and insoluble, iii) higher the DAC, higher the phosphate binding.

### Preparation of chewable compositions

For the purpose of the present invention, examples of the chewable compositions comprising a blend of the elastomers butyl rubber-polyisobutylene and chitosan were prepared by mixing the component without melting the same and by cold compression of the mixture in pieces of 520 mg or 1200 mg tablets. The chitosan used for the preparation of the chewable compositions is the chitosan coded as K2 in Table 1.

The contents of gum base as butyl rubber/polyisobutylene mixture were from 30 to 75 g/100 g and of the chitosan were from 20 to 100 mg.

The full details of the 4 chewable compositions prepared and the relative content (% by weight) of all ingredients are reported in Table 5.

**Table 5- Chewable compositions**

| **Component** | **#1** | | **#2** | | **#3** | | **#4** | |
|---|---|---|---|---|---|---|---|---|
| | **Amount (mg)** | **% (w/w)** | **Amount (mg)** | **%** | **Amount (mg)** | **%** | **Amount (mg)** | **% (w/w)** |
| **Butyl rubber- polyisobutylene** | 30 | 5.8 | 29 | 5.1 | 26 | 5.6 | 75 | 6.2 |
| Resins | 84 | 16.2 | 81 | 14.1 | 73 | 15.5 | 208 | 17.2 |
| Plasticizers | 67 | 12.9 | 64 | 11.2 | 58 | 12.3 | 165 | 13.7 |
| Sorbitol | 66 | 12.6 | 63 | 11.0 | 57 | 12.1 | 162 | 13.5 |
| Silicon dioxide | 9 | 1.7 | 9 | 1.6 | 8 | 1.6 | 33 | 2.7 |
| Talc | 58 | 11.2 | 55 | 9.7 | 51 | 10.7 | 143 | 11.9 |
| Isomalt | 141 | 27.1 | 135 | 23.6 | 123 | 25.9 | 347 | 28.8 |
| Magnesium stearate | 15 | 2.8 | 14 | 2.6 | 13 | 2.7 | 28 | 2.4 |
| Acesulfame K | 0.4 | 0.1 | 0.4 | 0.1 | 0.4 | 0.1 | 0.9 | 0.1 |
| Sodium saccharin | 0.3 | 0.1 | 0.3 | 0.1 | 0.3 | 0.1 | 0.4 | 0.1 |
| **Chitosan** | 40 | 7.7 | 60 | 19.2 | 100 | 11.5 | 20 | 1.7 |
| Flavour | 10 | 2.0 | 10 | 1.8 | 9 | 1.9 | 23 | 1.9 |

### Example 2: release of chitosan and phosphate binding capacity from the chewable composition 1

The release of chitosan from 6 sample of chewing gum of composition #1 of example 1 is detailed in Table 6. The release was assessed in a dissolution test using a commercially available chewing machine (DRT-2, ERWEKA). The test conditions were as follows: temperature: 37°C; chewing frequency: 30 strokes/min; chewing force: 8 bars; angle of rotation: 20°; distance between jaws: 2.0 mm; volume of the fluid in contact: 60 ml; maximum duration of exposure: 60 minutes. The test was conducted in three different mediums, simulating salivary normal composition (phosphate concentration: 0.28 g/L) at pH 6.5 and 7.4, and salivary hyperphosphoric composition (phosphate concentration: 2.24 g/L) at pH 7.4. The release was measured in the contact fluid from six samples at each time-point. The release of chitosan was measured spectrophotometrically at 570 nm after reaction of the sample with Cibacron Brilliant Red 3B-A (C.I. 18105) in 0.1 M Glycine buffer. No interference in the determination was seen from the other chewing gum components.

**Table 6- Chitosan release**

| Hyperphosphoric (H) simulated saliva, pH 7.4; normophosphoric (N) simulated saliva, pH 7.4; normophosphoric (N) simulated saliva, pH 6.5 | | | | | | | |
|---|---|---|---|---|---|---|---|
| **Medium** | **Time** | **Sample 1** | **Sample 2** | **Sample 3** | **Sample 4** | **Sample 5** | **Sample 6** |
| H pH 7.4 | 10 min | 0 mg | 0 mg | 0 mg | 1 mg | 1.1 mg | 0 mg |
| | 20 min | 0 mg | 1.3 mg | 0 mg | 0 mg | 0 mg | 0 mg |
| | 30 min | 0 mg | 0 mg | 4.4 mg | 0 mg | 0.0 mg | 0.4 mg |
| | 60 min | 0 mg | 0.5 mg | 0 mg | 0 mg | 0 mg | 0 mg |
| N pH 7.4 | 10 min | 0 mg | 0 mg | 0 mg | 0 mg | 0 mg | 0 mg |
| | 20 min | 0 mg | 0 mg | 0 mg | 0 mg | 0 mg | 0 mg |
| | 30 min | 0 mg | 0 mg | 0 mg | 0 mg | 0 mg | 0 mg |
| | 60 min | 0 mg | 0 mg | 0 mg | 0 mg | 0 mg | 0 mg |
| N pH 6.5 | 10 min | 0 mg | 0 mg | 0 mg | 0 mg | 0 mg | 0 mg |
| | 20 min | 0 mg | 0 mg | 0 mg | 0 mg | 0 mg | 0 mg |
| | 30 min | 0 mg | 0 mg | 0 mg | 1.3 mg | 0 mg | 0 mg |
| | 60 min | 0 mg | 0 mg | 0 mg | 0 mg | 0 mg | 0 mg |

As per Table 6, in different test conditions and at different time points, no chitosan was detected into the contact solutions, recovered from the chewing chamber. Minimal chitosan amounts were randomly recovered in some contact solutions, resulting from gum fragmentation due to forced, mechanical chewing stress. Therefore, no chitosan was directly released from the compositions of the present invention, demonstrating no release pattern by said compositions.

The phosphate binding capability of the compositions of the present invention was assessed into the chewed gums, recovered from the dissolution test in artificial saliva. The phosphate content of 6 samples (composition #1) for each time point (10, 20, 30 and 60 minutes) was measured after acid digestion of each sample in 12 ml aqua regia, at 95°C for 2 hours, with an ICP-AES at 178.287 nm. The phosphate binding was determined in three different mediums, simulating normophosphoric salivary composition (N) at pH 6.5 and 7.4, and hyperphosphoric salivary composition at pH 7.4. The results are summarized in Table 7a (mean±standard deviation), showing substantial phosphate removal from the medium in all the above reported conditions. As a proof of concept, Table 7b summarizes the results of the same test in a placebo composition made of the same gum base, but in the absence of chitosan. Results in Table 7b demonstrate the presence of some entrapment of phosphate into the gum matrix.

**Table 7a - P binding (composition)**

| Hyperphosphoric (H) simulated saliva, pH 7.4; normophosphoric (N) simulated saliva, pH 7.4; normophosphoric (N) simulated saliva, pH 6.5 | | | | |
|---|---|---|---|---|
| **Medium** | **10 min (mg PO₄)** | **20 min (mg PO₄)** | **30 min (mg PO₄)** | **60 min (mg PO₄)** |
| H pH 7.4 | 3 24 ± 0.61 | 2.71 ± 0.43 | 2.86 ± 0.25 | 2.78 ± 0.36 |
| N pH 7.4 | 2.25 ± 0.18 | 2.65 ± 0.13 | 2.32 ± 0.11 | 2.56 ± 0.13 |
| N pH 6.5 | 2.47 ± 0.11 | 2.99 ± 0.22 | 2.15 ± 0.12 | 2.74 ± 0.26 |

**Table 7b - P binding (placebo)**

| Hyperphosphoric (H) simulated saliva, pH 7.4; normophosphoric (N) simulated saliva, pH 7.4; normophosphoric (N) simulated saliva, pH 6.5 | | | | |
|---|---|---|---|---|
| **Medium** | **10 min (mg PO₄)** | **20 min (mg PO₄)** | **30 min (mg PO₄)** | **60 min (mg PO₄)** |
| H pH 7.4 | 1.01± 0.07 | 0.87± 0.10 | 0.90 ± 0.12 | 0.91 ± 0.25 |
| N pH 7.4 | 0.26 ± 0.02 | 0.33 ± 0.02 | 0.26 ± 0.03 | 0.31 ± 0.03 |
| N pH 6.5 | 0.31 ± 0.03 | 0.37 ± 0.03 | 0.25 ± 0.03 | 0.32 ± 0.04 |

Worth noting that the technical conditions required to dissolve the gum matrix in order to recover and measure the phosphates into the chewed gum are never found or reproduced in physiological conditions, as it could be the case of a chewed gum incidentally swallowed from a human being. The evidence of no release of chitosan from the compositions of the present invention, together with the P binding to the chitosan and stable entrapment of the phosphate into the compositions of the present invention, demonstrate that such compositions are the proper vehicle to stably remove salivary phosphate from intake.

### Example A.1: comparative examples of chewable compositions comprising polyisobutylene elastomer and chitosan

For the purpose of comparison, examples of compositions comprising polyisobutylene elastomer and chitosan, and the relative content (% w/w) of all the ingredients are reported in Table 8.

For the preparation of the composition the polyisobutylene elastomer was from 20 to 40 mg and the chitosan was 40 mg

The compositions were prepared by mixing the components and by cold compression in pieces of 520 mg and 1200 mg tablets. The chitosan employed is the sample K2 of Table 1.

**Table 8 - comparative chewable compositions**

| **Component** | **#5** | | **#6** | | **#7** | |
|---|---|---|---|---|---|---|
| | **Amount (mg)** | **%** | **Amount (mg)** | **%** | **Amount (mg)** | **%** |
| **Polyisobutylene** | 40 | 7.7 | 22 | 4.2 | 21 | 4.0 |
| Resins | 113 | 21.7 | 79 | 15.2 | 75 | 14.6 |
| Plasticizers | 71 | 13.6 | 59 | 11.3 | 57 | 10.9 |
| Sorbitol | 114 | 21.9 | 31 | 6.0 | 30 | 5.8 |
| Silicon dioxide | 7 | 1.3 | 10 | 1.9 | 10 | 1.9 |
| Talc | 31 | 5.9 | 8 | 1.6 | 8 | 1.6 |
| Isomalt | 78 | 15.0 | 282 | 54.1 | 270 | 51.9 |
| Sugar ester | 11 | 2.1 | 3 | 0.5 | 3 | 0.5 |
| Acesulfame K | 0,6 | 0.1 | 0.6 | 0.1 | 0.6 | 0.1 |
| **Chitosan** | 40 | 7.7 | 20 | 3.9 | 40 | 7.7 |
| Flavour | 15 | 2.9 | 5 | 1.0 | 5 | 1.0 |

### Example A. 2: release of chitosan and phosphate binding capacity from the comparative chewable compositions 5 and 7

The release of chitosan from compositions #5 and #7 in comparison with the chewable composition #1 of example 1 is detailed in Table 9a, whereas the P binding is reported in Table 9b. The performances of these example compositions, when compared with the preferred compositions of the present invention, demonstrate that the chitosan is not released, but P binding by chitosan occurs to a very limited extent, i.e. in the order of 5 to 9 times less. Further, as a proof of concept, Table 9c summarizes the results of the same test in a placebo composition made of polyisobutylene but in the absence of chitosan. In all tests hyperphosphoric simulated saliva, pH 7.4 was used.

**Table 9a - chitosan release**

| **Comp.** | **Time** | **Sample 1** | **Sample 2** | **Sample 3** | **Sample 4** | **Sample 5** | **Sample 6** |
|---|---|---|---|---|---|---|---|
| #1 | 15 min | 0 mg | 0 mg | 0 mg | 0 mg | 0 mg | 0 mg |
| | 30 min | 0 mg | 0 mg | 0 mg | 0 mg | 0 mg | 0 mg |
| | 60 min | 0 mg | 0 mg | 0 mg | 0 mg | 0 mg | 0 mg |
| #5 | 15 min | 0 mg | 0 mg | 0 mg | 0 mg | 0 mg | 0 mg |
| | 30 min | 0 mg | 0 mg | 0 mg | 0 mg | 0 mg | 0 mg |
| | 60 min | 0 mg | 0 mg | 0 mg | 0 mg | 0 mg | 0 mg |
| #7 | 15 min | 0 mg | 0 mg | 0 mg | 0 mg | 0 mg | 0 mg |
| | 30 min | 0 mg | 0 mg | 0 mg | 0 mg | 0 mg | 0 mg |
| | 60 min | 0 mg | 0 mg | 0 mg | 0 mg | 0 mg | 0 mg |

| | | | | | | | |
|---|---|---|---|---|---|---|---|
| #1 preferred embodiment; #5 and #7 comparative examples | | | | | | | |

**Table 9b - P binding (comparison of compositions)**

| **composition** | **15 min (mg PO₄)** | **30 min (mg PO₄**) | **60 min (mg PO₄)** |
|---|---|---|---|
| **#1** | 2.76 ± 0.18 | 2.17 ± 0.22 | 2.65 ± 0.28 |
| **#5** | 0.88 ± 0.10 | 0.95 ± 0.07 | 0.96 ± 0.14 |
| **#7** | 0.72 ± 0.12 | 0.59 ± 0.10 | 0.77 ± 0.11 |

| | | | |
|---|---|---|---|
| #1 preferred embodiment; #5 and #7 negative examples | | | |

**Table 9c P binding (placebos)**

| **Sample** | **15 min (mg PO₄)** | **30 min (mg PO₄)** | **60 min (mg PO₄)** |
|---|---|---|---|
| **#1** | 0.74 ± 0.05 | 0.52 ± 0.16 | 0.68 ± 0.13 |
| **#5** | 0.62 ± 0.14 | 0.46 ± 0.12 | 0.64 ± 0.17 |
| **#7** | 0.75 ± 0.12 | 0.69 ± 0.12 | 0.76 ± 0.09 |

| | | | |
|---|---|---|---|
| #1 preferred embodiment; #5 and #7 negative examples | | | |

### Example 3: effect of salivary phosphate binding in Chronic Kidney Disease

Two clinical efficacy studies: a double-blind placebo controlled trial in patients with ESRD (Efficacy Study A) and an open-label trial in those with stage 3-4 CKD (Efficacy Study B) were conducted.

The chewable compositions used for the studies were as per compositions 1, 2 and 4 of example 1.

### Efficacy Study A

The study was an 8 week double-blind, placebo-controlled randomized clinical trial of chitosan containing chewing gum in 92 subjects with end-stage renal disease on hemodialysis conducted at a single center. Key inclusion criteria included overt hyperphosphatemia, as per a mean historical or mean screening serum P ≥ 4.7 and ≤ 9 mg/dL, URR > 65%, stable doses of intestinal P binders, vitamin D analogs or calcimimetics, and a screening Saxon test (a measure of the amount of saliva produced over 2 minutes) of at least 1.2g. Key exclusion criteria included a sensitivity to chitin or shellfish, recent infection or hospitalization, inability to chew for 60 minutes, and any unstable medical condition which might compromise successful completion of the study protocol. Subjects were randomized 1:1:1 to receive either 20 mg chitosan gum as per composition 4, 40 mg chitosan gum as per composition 1, or matching placebo. The study design included a 4 week double blind chewing period (twice daily for all subjects), a 4 week washout period and a 2 week open label period in which all subjects were given 40 mg chitosan gum as per composition 1, 3 times daily. The primary endpoint was the change in serum P from baseline to day 29 of active gum chewing. No changes in intestinal P binders, vitamin D analogs, oral nutritional vitamin D, calcium supplements, calcimimetics or dialysis Rx were allowed during the trial. Serum chemistries were assessed using standard auto-analyzer techniques (Quest Laboratories, Denver CO).

### Results

92 subjects were randomized (n=30 placebo, n=31 20 mg chitosan chewing gum of composition 4, n=31 40 mg chitosan chewing gum as per composition 1) and all were included in the ITT analysis. 3 subjects were noncompliant with study medication, 1 subject had a change in vitamin D dose and 1 subject was removed for administrative reasons. All were included in the safety population. Overall compliance with gum chewing was assessed by collection of chewed gum and was > 95% among all treatment arms. Baseline serum P was 2.03 mmol/L (i.e. 6.3 mg/dL by the conversion factor: mmol/L divided by 0.32) in all groups. Serum P was unchanged at day 29 in placebo treated subjects and reduced by a mean of 0.2 mg/dL in actively treated subjects (p=0.39). At end of washout, average serum P was 6.5 mg/dL and 6.2 mg/dL in placebo/actively treated patients. All patients received open-label 20 mg chitosan chewing gum as per composition 4 thrice daily during the final 2 weeks of study. At the end of the open label phase, mean serum P was reduced by 0.2 mg/dL in all subjects, including those previously receiving placebo in the randomized phase of the efficacy study (p=0.03 versus end of washout).

The well-known healthy, normal averaged salivary P content is 8-10 mg/dL. The mean baseline salivary P was 26.25 mg/dL in placebo subjects and 23.12 mg/dL in actively treated subjects, i.e salivary P was increased in CKD, dialysis patients

### Efficacy Study B

The study was a multicenter 4 week open-label randomized clinical trial of 20 mg chitosan chewing gum as per composition 4, in 147 patients with CKD not on dialysis having an estimated GFR (eGFR) 20-45 ml/min/m² with a serum P ≥ 3.53 mg/dL and a Saxon test > 1 g. Mean salivary P was 20.3 mg/dL, i.e. increased in comparison with the well-known healthy, normal levels. After a 2 week run-in period, all subjects entered a 2 week open label active chewing period during which they chewed gum thrice daily for 30 minutes. Subjects were deemed 'early responders' if they had a reduction from baseline in their serum P of at least 0.2 mg/dL during active chewing or during the 1^{st} week of a subsequent washout period. All early responders then completed a total of 2 weeks of washout before being randomized to either receive 60 mg chitosan chewing gum as per composition 2, thrice daily or to remain off chewing gum and were followed for an additional 2 weeks. Study inclusion and exclusion criteria and prohibited medication use was similar to efficacy study A. The protocol required that study visits were conducted in the afternoon and saliva was collected in a passive manner based on the results of pilot investigations. Serum chemistries were assessed using standard auto-analyzer techniques (Quest Laboratories, Denver CO). The primary objective of the study was to assess the change in serum P from baseline to end of active treatment while a key secondary endpoint was the difference in serum P during the 2 week randomized period in early responders between those chewing gum and those remaining off chewing gum.

### Results

One-hundred fifty-seven subjects were enrolled and 150 attended the day 1 visit and are included in safety analyses. Of these, 147 returned for subsequent visits and are included in the analyses of efficacy. Seven subjects did not return for the day 29 visit. Of the 140 subjects who attended the day 22 and day 29 visits, 69 were considered to be 'early responders' and were randomized to either chew chitosan gum for an additional 2 weeks or to remain off of study medication. The subjects' average age was 66 years, 58% were male, 70% were Caucasian, and the average duration of CKD was 60 months (IQR 29-78 months). The average eGFR was 34 ml/min (IQR 22-44) and the average baseline serum P was 3.97 mg/dL. There were no statistically significant differences between early responders and non-responders in any baseline demographic or laboratory parameters with the exception that all 6 subjects with a history of 'dry mouth' were in the early responder group (p=0.009).

During the open-label active treatment phase serum P was reduced by 0.16 mg/dL at day 15 (p=0.003 versus day 1). Sixty-nine subjects were classified as 'early responders' on the basis of a decrease of serum P by at least 0.2 mg/dL. Among these subjects, those randomized to remain off of study medication experienced an increase in serum P of 0.2 mg/dL while those randomized to thrice daily chitosan gum had a decrease in serum P of 0.1 mg/dL (p=0.03 for comparison of change between treatment versus no treatment, p=ns for within arm change from washout in each group).

Mean salivary P was 20.3 mg/dL at baseline and there was no statistically significant change at any time point.

Using an a priori definition of meaningful change in serum P of 0.2 mg/dL, a *post* hoc analysis was conducted to evaluate if there were significant differences with treatment on this outcome. At the end of the open-label active treatment period (day 15) 50% of subjects had experienced a decrease in serum P of at least 0.2 mg/dL while 27% of subjects had experienced an equivalent increase (p=0.002). Similarly, during the randomized treatment period for early responders, 46% of those assigned to chewing gum experienced a decrease in serum P of at least 0.2 mg/dL while only 18% of those assigned to *no chewing* had a similar decrease (p=0.03).

### Conclusions

Twice daily 20 mg chitosan gum as per composition 4 over 4 weeks reduced serum phosphate by 0.2 mg/dL in the double-blind placebo controlled trial in ESRD (p=ns versus placebo). In an open label extension in these subjects, 40 mg chitosan gum as per composition 1 thrice daily reduced serum phosphate by 0.2 mg/dL (p=0.03 versus end of washout). In a 2 week open-label trial in patients with CKD not on dialysis, 20 mg chitosan gum as per composition 4 given TID reduced serum phosphate by 0.05 mmol/L (p=0.003 versus day 1).

## Claims

1. A chewable composition comprising a gum base consisting of synthetic elastomers and mixtures thereof and a chitosan, wherein the chitosan has a viscosity (in 1% acetic acid) in the range from 10 to 100 mPa.s and a deacetylation degree (DAC) of not less than 90% and is retained and/or not released from the composition and is capable to bind salivary phosphorous compounds by up-taking the same from saliva *in situ,* for use to reduce serum levels of phosphorous compounds in disorders of phosphorous mineral metabolism.

2. The chewable composition for use according to claim 1, wherein the phosphorous compounds or the phosphorous compounds-chitosan complexes formed are entrapped into said composition from where they are not dissolved and released.

3. The chewable composition for use according to claim 1, wherein the ratio between the gum base and the chitosan is in the range from 1:4 to 4:1 by weight.

4. The chewable composition for use according to claim 1, wherein the ratio between the gum base and the chitosan is 1:1 by weight.

5. The chewable composition for use according to claim 1, wherein the amount of the gum base is from 5 to 6 % by weight.

6. The chewable composition for use according to claim 1, wherein the amount of the chitosan 1.5 to 20.0 % by weight.

7. The chewable composition for use according to claim 1, wherein the chitosan is selected in the group of animal or vegetal chitosans.

8. The chewable composition for use according to claim 1, wherein the chitosan has a viscosity from 70 to 90 mPa.s.

9. The chewable composition for use according to claim 1, wherein the chitosan has a deacetylation degree (DAC) equal or higher than 95%.

10. The chewable composition for use according to claim 1, wherein the gum base is a mixture of synthetic elastomers selected from isobutylene-isoprene copolymers, polyisobutylene, polyisoprene, styrene-butadiene copolymers, polyethylene, polyvinyl acetate, polyvinyl acetate/laurate copolymers and mixtures thereof.

11. The chewable composition for use according to claim 10, wherein the gum base is a mixture of synthetic elastomers comprising at least butyl rubber (isobutylene-isoprene copolymers) and polyisobutylene polymers.

12. The chewable composition for use according to claim 10, wherein the gum base is a mixture of butyl rubber (isobutylene-isoprene copolymers) and polyisobutylene polymers.

13. The chewable composition for use according to one of the claims from 1 to 12 further comprising components selected from plasticizers, resins, anti-oxidants, sweeteners, excipients and flavours with the provision that said components do not interfere on oral pH by acidification.

14. The chewable composition for use according to claim 13, wherein the plasticizers is selected from vegetal natural rubbers.

15. The chewable composition for use according to claim 1, wherein the composition is for use in the treatment of phosphorous metabolism disorders associated with a detectable increased salivary phosphate content.

16. The chewable composition for use according to one of the claims 1 or 15, wherein said disorders are selected from the group consisting of early and advanced stages of chronic kidney disease, diabetes, hypertension, cardio-vascular diseases **characterized by** extra osseus calcification of the tunica media, mineral bone disorders and hyperparathyroidism.

## Patentansprüche

1. Kaubare Zusammensetzung, umfassend eine Gummibasis, bestehend aus synthetischen Elastomeren und Gemischen davon und einem Chitosan, wobei das Chitosan eine Viskosität (in 1 % Essigsäure) im Bereich von 10 bis 100 mPa.s und einen Deacetylierungsgrad (DAC) von mindestens 90 % aufweist und von der Zusammensetzung zurückgehalten und/oder nicht aus ihr freigesetzt wird und in der Lage ist, speichernde Phosphorverbindungen durch Aufnehmen derselben aus Speichel in situ zu binden, zur Verwendung zur Reduzierung des Serumspiegels von Phosphorverbindungen bei Störungen des Phosphormineralmetabolismus.

2. Kaubare Zusammensetzung zur Verwendung nach Anspruch 1, wobei die Phosphorverbindungen oder die gebildeten Phosphorverbindungs-Chitosankomplexe in die Zusammensetzung eingeschlossen sind, aus denen sie nicht gelöst und freigegeben werden.

3. Kaubare Zusammensetzung zur Verwendung nach Anspruch 1, wobei das Verhältnis zwischen der Gummibasis und dem Chitosan im Bereich von 1:4 bis 4:1, bezogen auf die Masse, liegt.

4. Kaubare Zusammensetzung zur Verwendung nach Anspruch 1, wobei das Verhältnis zwischen der Gummibasis und dem Chitosan 1:1, bezogen auf die Masse, beträgt.

5. Kaubare Zusammensetzung zur Verwendung nach Anspruch 1, wobei die Menge der Gummibasis 5 bis 6 % Massenanteil beträgt.

6. Kaubare Zusammensetzung zur Verwendung nach Anspruch 1, wobei die Menge des Chitosans 1,5 bis 20,0 % Massenanteil beträgt.

7. Kaubare Zusammensetzung zur Verwendung nach Anspruch 1, wobei das Chitosan in der Gruppe der tierischen oder pflanzlichen Chitosane ausgewählt ist.

8. Kaubare Zusammensetzung zur Verwendung nach Anspruch 1, wobei das Chitosan eine Viskosität von 70 bis 90 mPa.s aufweist.

9. Kaubare Zusammensetzung zur Verwendung nach Anspruch 1, wobei das Chitosan einen Deacetylierungsgrad (DAC) von gleich oder höher als 95 % aufweist.

10. Kaubare Zusammensetzung zur Verwendung nach Anspruch 1, wobei die Gummibasis eine Mischung aus synthetischen Elastomeren ist, ausgewählt aus Isobutylen-Isopren-Copolymeren, Polyisobutylen, Polyisopren, Styrol-Butadien-Copolymeren, Polyethylen, Polyvinylacetat, Polyvinylacetat-/Laurat-Copolymeren und Mischungen davon.

11. Kaubare Zusammensetzung zur Verwendung nach Anspruch 10, wobei die Gummibasis eine Mischung aus synthetischen Elastomeren ist, die mindestens Butylkautschuk (Isobutylen-Isopren-Copolymere) und Polyisobutylenpolymere umfasst.

12. Kaubare Zusammensetzung zur Verwendung nach Anspruch 10, wobei die Gummibasis eine Mischung aus Butylkautschuk (Isobutylen-Isopren-Copolymere) und Polyisobutylenpolymeren ist.

13. Kaubare Zusammensetzung zur Verwendung nach einem der Ansprüche von 1 bis 12, ferner umfassend Komponenten, ausgewählt aus Weichmachern, Harzen, Antioxidantien, Süßungsmitteln, Hilfsstoffen und Aromen, mit der Maßgabe, dass die Komponenten den oralen pH-Wert nicht durch Versauerung stören.

14. Kaubare Zusammensetzung zur Verwendung nach Anspruch 13, wobei die Weichmacher ausgewählt sind aus pflanzlichen Naturkautschuken.

15. Kaubare Zusammensetzung zur Verwendung nach Anspruch 1, wobei die Zusammensetzung zur Verwendung bei der Behandlung von Störungen des Phosphormetabolismus bestimmt ist, die mit einem nachweisbaren erhöhten Phosphatgehalt im Speichel verbunden sind.

16. Kaubare Zusammensetzung zur Verwendung nach einem der Ansprüche 1 oder 15, wobei die Störungen aus der Gruppe bestehend aus frühen und fortgeschrittenen Stadien von chronischer Nierenerkrankung, Diabetes, Bluthochdruck, Herz-Kreislauf-Erkrankungen ausgewählt sind, **gekennzeichnet durch** zusätzliche osseale Verkalkung der Tunica media, mineralische Knochenerkrankungen und Hyperparathyreoidismus.

## Revendications

1. Composition à mâcher comprenant une base de gomme composée d'élastomères synthétiques et de mélanges de ceux-ci et de chitosane, dans laquelle le chitosane présente une viscosité (dans l'acide acétique à 1%) allant de 10 à 100 mPa.s et un degré de désacétylation (DAC) non inférieur à 90% et est retenu et/ou non libéré de la composition et est capable de se lier aux composés phosphorés de la salive, en les utilisant à partir de la salive *in situ*, pour réduire les taux sériques des composés phosphorés dans les troubles du métabolisme des minéraux phosphorés.

2. Composition à mâcher à utiliser selon la revendication 1, dans laquelle les composés phosphorés ou les complexes composés phosphorés-chitosane formés sont piégés dans ladite composition à partir de laquelle ils ne sont pas dissous et libérés.

3. Composition à mâcher à utiliser selon la revendication 1, dans laquelle le rapport entre la base de gomme et le chitosane est dans l'intervalle 1:4 à 4:1 en poids.

4. Composition à mâcher utilisée selon la revendication 1, dans laquelle le rapport entre la base de gomme et le chitosane est 1:1 en poids.

5. Composition à mâcher utilisée selon la revendication 1, dans laquelle la quantité de la base de gomme est de 5 à 6% en poids.

6. Composition à mâcher utilisée selon la revendication 1, dans laquelle la quantité de chitosane est de 1,5 à 20,0% en poids.

7. Composition à mâcher utilisée selon la revendication 1, dans laquelle le chitosane est sélectionné dans le groupe des chitosanes d'origine animale ou végétale.

8. Composition à mâcher utilisée selon la revendication 1, dans laquelle le chitosane présente une viscosité de 70 à 90 mPa.s.

9. Composition à mâcher utilisée selon la revendication 1, dans laquelle le chitosane a un degré de désacétylation (DAC) égal ou supérieur à 95%.

10. Composition à mâcher à utiliser selon la revendication 1, dans laquelle la base de gomme est un mélange d'élastomères synthétiques sélectionnés parmi les copolymères isobutylène-isoprène, polyisobutylène, polyisoprène, les copolymères styrène-butadiène, polyéthylène, acétate de polyvinyle, les copolymères acétate de polyvinyle/laurate et leurs mélanges.

11. Composition à mâcher à utiliser selon la revendication 10, dans laquelle la base de gomme est un mélange d'élastomères synthétiques comprenant au moins du caoutchouc butyle (copolymères d'isobutylène-isoprène) et de polymères de polyisobutylène.

12. Composition à mâcher à utiliser selon la revendication 10, dans laquelle la base de gomme est un mélange de caoutchouc butyle (copolymères d'isobutylène-isoprène) et de polymères de polyisobutylène.

13. Composition à mâcher à utiliser selon l'une des revendications 1 à 12, comprenant en outre des composants sélectionnés parmi les plastifiants, les résines, les anti-oxydants, les édulcorants, les excipients et les arômes, à condition que lesdits composants n'interfèrent pas sur le pH oral par acidification.

14. Composition à mâcher à utiliser selon la revendication 13, dans laquelle les plastifiants sont sélectionnés parmi les caoutchoucs naturels végétaux.

15. Composition à mâcher à utiliser selon la revendication 1, dans laquelle la composition est utilisée dans le traitement des troubles du métabolisme du phosphore, associés à une augmentation détectable de la teneur en phosphate de la salive.

16. Composition à mâcher à utiliser selon l'une des revendications 1 ou 15, dans laquelle lesdits troubles sont sélectionnés dans le groupe comprenant les stades précoces et avancés de la maladie rénale chronique, du diabète, de l'hypertension, des maladies cardiovasculaires **caractérisées par** une calcification extra osseuse de la tunique moyenne, des troubles osseux et minéraux et de l'hyperparathyroïdie.
